# EUROPEAN PATENT APPLICATION

(11) **EP 2 123 254 A1**
(43) Date of publication of application: **25.11.2009**
(21) Application number: 08722511.6
(22) Date of filing: 19.03.2008
(51) Int. Cl.: A61K 8/44, A61K 31/197, A61P 17/16, A61Q 1/02, A61Q 1/12, A61Q 19/00, A61Q 19/08

(54) **COMPOSITION FOR EXTERNAL APPLICATION TO SKIN**

(30) Priority: 20.03.2007 JP 2007073240
(71) Applicant: Shiseido Company, Ltd., Chuo-ku Tokyo 104-8010 (JP)
(72) Inventor: Tsunenaga, Makoto, Yokohama-shi Kanagawa 224-8558 (JP); Ochiai, Nobuhiko, Yokohama-shi Kanagawa 224-8558 (JP); Kaminuma, Mikiko, Yokohama-shi Kanagawa 224-8558 (JP); Suetsugu, Masaru, Yokohama-shi Kanagawa 224-8558 (JP)
(74) Representative: Hart-Davis, Jason
(86) International application number: PCT/JP2008/055136
(87) International publication number: WO 2008/126652

(57) **Abstract**

A moisturizing agent comprising one or more than one compound selected from the group consisting of lysyl-β-alanine represented by the following general formula (1) and a salt thereof:

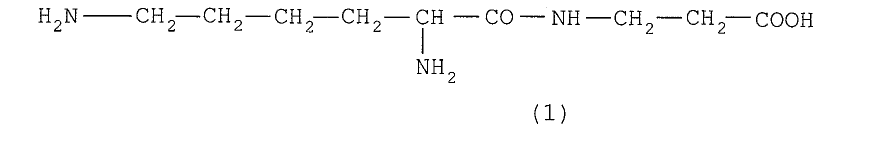

## Description

### FIELD OF THE INVENTION

The present invention relates to an external composition for skin.

More specifically the present invention relates to: a moisturizing agent that has an excellent safety, an excellent sense of use, and an excellent permeability, acts directly on the horny layer to increase the amount of water in the horny layer and has an excellent persistent moisturizing effect; and an external composition for skin having an excellent effect of retaining moisture, preventing and improving wrinkles that acts directly on the horny layer to increase water in the horny layer, has an excellent safety, an excellent stability and an excellent sense of use.

### BACKGROUND ART

Aging proceeds in all organs of the body. With regard to the skin which is visible among them, specifically the face on which attention tends to be concentrated, wrinkles and fine lines that develop with aging are annoying many middle-aged and older people, specifically women. In response to a strong need for a wrinkle-improving cosmetic, conventionally various attempts to address the need have been made. No inventions with a satisfactory effect have been made so far, however, since much of the mechanism related to aging and wrinkles is unknown. Under such circumstances, on the other hand, increasing and maintaining water in the outermost horny layer of the epidermis has been expected to serve a certain purpose since it could keep the skin flexible and elastic and protect the dermis.

Conventionally, a moisturizing agent has been blended in external compositions for skins for the purpose of enhanced sense of use and safety and disinfection in addition to a moisturizing effect. There can be mentioned, for example, polyols such as glycerin, 1,3-butyleneglycol, xylitol, sorbitol, erythritol, maltitol, polyethylene glycol, propylene glycol, diglycerin (EO) (PO) adducts, and polyethers. Though they have a moisturizing effect, they do not have sufficient effects of preventing and improving wrinkles, and they had problems of stickiness and sense of use, and the development of odors due to oxidation.

Also, polymer compounds such as hyaluronic acid, mucoitinsulfuric acid, chondroitin sulfate, soluble collagen, and atelocollagen have been used as a moisturizing agent. Though they have a high moisturizing effect compared with polyols, the moisturizing effect not sufficient to prevent and improve wrinkles. Furthermore, the effect is to moisturize the environment around the skin, the permeability of polymer compounds into the skin is poor and they do not act directly on the skin. They also had problems of cumbersome process of neutralization and dissolution during blending, and problems at use such as a taut feeling due to a coated feeling and a higher stickiness than polyols.

As low molecular weight compounds other than polyols, sodium lactate, bile acid salts, pyrrolidonei carboxylate, and amino acids are also widely used as moisturizing agents. Though, being low molecular weight, they are expected to permeate into the horny layer and function themselves, their moisturizing effect is not high. Furthermore, they are highly crystalline and poorly soluble in water, and thus they had problems that, when blended with an external composition for skin, they tend to crystallize, after application, with the evaporation of water, they have a poor ability of persistently retaining water, and their permeation into the horny layer is suppressed. Also, specifically many amino acids are organoleptic, and for example the amount blended of glycine, β-alanine, γ-aminobutyric acid (GABA) etc. has been limited since they may produce skin irritancy at application on users having the sensitive skin. There are also problems of odors: e.g. low molecular weight basic amino acids such as lysine may produce an amine odor, and thus in external preparations for skin likely to be applied on the face near the nose, their amount to be blended therein has been limited.

After intensive and extensive research on a substance that has a high moisturizing effect, an excellent persistent moisturizing ability, no organoleptic or odor problems, high safety and an excellent permeability, and that acts directly on the horny layer to increase water content in the horny layer, and an external composition for skin having the substance blended therein has an excellent safety considering the above circumstances, the present inventor has found that lysyl-β-alanine and its salt of the present invention is a substance that has a high moisturizing effect, an excellent persistence of moisturizing effect, and high permeability, and acts directly on the horny layer to increase water content in the horny layer, has no organoleptic at application, and is highly safe, has no odor problems, and the preparation of an external composition for skin having it blended therein is easy, and the external composition for skin having it blended therein has an excellent safety and excellent sense of use, and therefore has completed the present invention.
Patent document 1: Japanese Examined Patent Publication (Kokoku) No. 8-11746.

### DISCLOSURE OF THE INVENTION

### PROBLEMS TO BE SOLVED BY THE INVENTION

The present invention intends to provide: a moisturizing agent that has an excellent safety, an excellent usability, and an excellent permeability while having no organoleptic or order problems, acts directly on the horny layer to increase the amount of water in the horny layer, and an excellent persistent moisturizing effect; and an external composition for skin that acts directly on the horny layer to increase water in the horny layer, has an excellent persistent moisturizing effect, no organoleptic or odor problems, an excellent safety and stability, an effect of retaining moisture, preventing and improving wrinkles accompanied by an excellent sense of use.

### MEANS TO SOLVE THE PROBLEMS

Thus, the present invention intends to provide a moisturizing agent comprising one or more than one compound selected from the group consisting of lysyl-β-alanine represented by the following general formula (1) and a salt thereof:

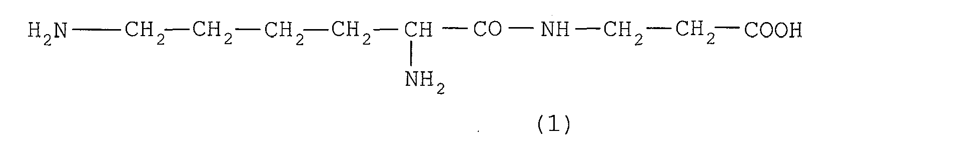

The present invention also intends to provide an external composition for skin that acts directly on the horny layer to increase water in the horny layer, has an excellent persistence of moisturizing effect, no organoleptic or odor problems, an excellent safety and stability, an effect of retaining moisture, preventing and improving wrinkles accompanied by an excellent sense of use, said composition comprising a compound selected from the group consisting of lysyl-β-alanine represented by the following general formula (1) and a salt thereof.

### EFFECT OF THE INVENTION

In accordance with the present invention, there can be provided: a moisturizing agent that has an excellent moisturizing effect, an excellent persistence of moisturizing effect, no organoleptic or odor problems, an excellent safety, an excellent sense of use, and an excellent skin permeability, acts directly on the horny layer to increase the amount of water in the horny layer, and an excellent persistent moisturizing effect; and an external composition for skin that has an excellent skin permeability, acts directly on the horny layer to increase the amount of water in the horny layer, has an excellent persistence of moisturizing effect, an excellent safety, an excellent stability, no organoleptic or odor problems, and an effect of retaining moisture, preventing and improving wrinkles accompanied by an excellent sense of use.

### BEST MODE FOR CARRYING OUT THE INVENTION

The moisturizing agent of the present invention comprises one or more than one lysyl-β-alanine represented by the following general formula (1) or a salt thereof as an active component having a moisturizing effect. The external composition for skin of the present invention also comprises one or more than one lysyl-β-alanine represented by the following general formula (1) or a salt thereof.

Lysyl-β-alanine represented by the following general formula (1) for use in the present invention is a known compound used in a chemical name such as N-(2,6-diamino-hexanoyl)-2-aminopropionic acid. It is a dipeptide formed of lysine and β-alanine. For lysine, it may be a L form, a D form, or a DL form, representing L-lysyl-β-alanine, D-lysyl-β-alanine, or D,L-lysyl-β-alanine, respectively. In accordance with the present invention, it may take any form, and any composition ratio in the DL-form.

Lysyl-β-alanine is known for its use as a central nervous system drug, but its moisturizing effect of the present invention is novel and its use in external compositions for skin is also novel (Patent document 1).

Lysyl-β-alanine for use in the present invention may be easily synthesized by a known method, or may be extracted from natural sources, or obtained by an enzymatic method etc.

As salts of lysyl-β-alanine represented by the general formula (1), there can be mentioned, but not limited to, a hydrochloride, a sulfate, a phosphate, a hydrobromide, an alkylsulfate such as a methyl sulfate and a p-toluene sulfonate, an acid salt such as an acetate, a lactate, a malate, a fumarate, an oxalate, a succinate, a tartrate, and a citrate, a salt with an amino acid such as a betaine salt, a glycine salt, an alanine salt, a serine salt, a taurine salt, a glutamate, and an aspartate, a sodium salt, a potassium salt, a magnesium salt, a calcium salt, an ammonium salt, a triethanolamine salt, and a diethanolamine salt. The ratio of lysyl-β-alanine to a counter ion that constitutes a salt is not specifically limited, and a plurality of counter ions may be combined.

In accordance with the present invention, the amount blended of lysyl-β-alanine represented by the general formula (1) or a salt thereof may be 0.001-20% by weight, preferably 0.1-10.0% by weight in the total amount of the moisturizing agent or the external composition for skin. At 0.001% by weight or less, the effect may be poor, and the blending of an amount exceeding 20.0% by weight may not give an enhanced effect.

The composition claimed in the present invention may be produced according to a conventional method, and, as an component constituting the composition, one or more than one lysyl-β-alanine represented by the general formula (1) or a salt thereof may be used alone, or may be blended, as appropriate, with a component used in external preparations for skin etc. for cosmetics normally including quasi-drugs and pharmaceuticals, for example, an oil, a surfactant, a powder, a dye, water, an alcohol, a thickening agent, a chelating agent, a silicone, an antioxidant, a UV-absorber, a moisturizing agent, a perfume, various pharmaceutical components, a disinfectant, a pH-adjusting agent, and a neutralizing agent.

As an oil among the above arbitrary blending components, there can be mentioned a higher alcohol such as a liner-chain alcohol such as lauryl alcohol, cetyl alcohol, stearyl alcohol, myristyl alcohol, and oleyl alcohol, and a branched-chain alcohol such as monostearyl glycerin ether, lanoline alcohol, cholesterol, phytosterol, and isostearyl alcohol, a higher fatty acid such as lauric acid, myristic acid, palmitic acid, and a stearic acid, a wax such as solid paraffin, beeswax, hydrogenated castor oil, carnauba wax, and Bareco wax, an animal and plant oil such as beef tallow, lard, mutton tallow, squalane, coconut oil, palm oil, palm kernel oil, soybean oil, olive oil, cotton seed oil, jojoba oil, castor oil, and lanoline, a mineral oil such as liquid paraffin and petrolatum, a synthetic oil such as trimethylpropane triisostearate, isopropyl myristate, glycerol tri-2-ethyl hexanoate, pentaerythritol tetra-2-ethyl hexanoate, silicone oil, and polyoxyethylene (hereinafter referred to as POE) polyoxypropylene (hereinafter referred to as POP) pentaerythritol ether.

As surfactants, there can be mentioned fatty acid soaps such as soap base, sodium laurate and sodium palmitate, higher alkylsulfuric ester salts such as sodium laurylsulfate and potassium laurylsulfate, alkyl ether sulfuric ester salts such as triethanolamine POE laurylsulfate and sodium POE laurylsulfate, N-acylsarcosine acids such as sodium lauroylsarcosinate, higher fatty acid amide sulfonates such as sodium N-myristoyl-N-methyltaurate and sodium methyltaurate cocoate, phosphoric ester salts such as POE stearyl ether phosphoric acid, sulfosuccinates such as sodium monolauroyl monoethanolamide POE sulfosuccinate and sodium laurylpolypropylene glycol sulfosuccinate, alkylbenzenesulfonates such as sodium linear dodecylbenzenesulfonate and triethanolamine linear dodecylbenzenesulfonate, N-acylglutamates such as disodium N-stearoylglutamate and monosodium N-stearoylglutamate, higher fatty acid ester sulfates such as sodium hydrogenated glyceryl cocoate sulfate, sulfated oils such as turkey red oil, as well as POE alkyl ether carboxylate, POE alkylallyl ether carboxylate, higher fatty acid ester sulfonate, sec-alcohol sulfate, higher fatty acid alkyloyl amide sulfate, sodium lauroyl monoethanolamine succinate, and anionic surfactants such as casein sodium;
cationic surfactants including alkyltrimethyl ammonium salts such as stearyltrimethyl ammonium chloride and lauryltrimethyl ammonium chloride, dialkyl dimethyl ammonium salts such as distearyl dimethyl ammonium chloride, alkylpyridinium salts such as cetyl pyridinium chloride, as well as alkyl quarternary ammonium salt, alkyldimethylbenzyl ammonium salt, alkylisoquinolinium salt, dialkylmorphonium salt, POE alkylamine, alkylamine salt, polyamine fatty acid derivatives, amylalcohol fatty acid derivatives, and benzalkonium chloride; ampholytic surfactants including imidazoline type ampholytic surfactants such as 2-cocoyl-2-imidazalinium hydroxide-1-carboxyethyloxy disodium salt, and betaine type surfactants such as amide betaine and sulfobetaine; lipophilic non-ionic surfactants including sorbitan fatty acid esters such as sorbitan monooleate, sorbitan monoisostearate, sorbitan monolaurate, sorbitan monopalmitate, and sorbitan trioleate, glycerin polyglycerin fatty acids such as mono-cottonseed-fatty acid glycerin, glyceryl monostearate, glyceryl sesquioleate, and glyceryl monostearate malate, propylene glycol fatty acid esters such as propylene glycol monostearate, as well as hydrogenated castor oil derivatives, glycerol alkyl ether, hydrophilic non-ionic surfactants such as POE-methylpolysiloxane copolymers; hydrophilic nonionic surfactants including POE sorbitan fatty acid esters such as POE-sorbitan monooleate and POE-sorbitan monostearate, POE-sorbitol fatty acid esters such as POE-sorbitol monolaurate, POE-sorbitol monooleate and POE-sorbitol monostearate, POE-glycerol fatty acid esters such as POE-glyceryl monostearate and POE-glyceryl distearate, POE fatty acid esters such as POE monooleate, POE distearate, and POE monodioleate, POE alkyl ethers such as POE lauryl ether, POE oleyl ether and POE cholestanol ester, POE alkyl phenyl ethers such as POE octyl phenyl ether and POE nonyl phenyl ether, pluaronics such as pluronic, POE-POP alkyl ethers such as POE-POP monobutyl ether, POE-POP cetyl ether and POE-POP glycerol ether, POE castor oil hydrogenated castor oil derivatives such as POE castor oil, POE hydrogenated castor oil, POE hydrogenated castor oil monoisostearate and POE hydrogenated castor oil maleate, POE bees wax laurin derivatives such as POE sorbitol bees wax, alkanol amides such as coconut fatty acid diethanol amide and fatty acid isopropanol amide, as well as POE propylene glycol fatty acid ester, POE fatty acid amide, POE alkyl amine, sucrose fatty acid ester, and alkylethoxydimethylamine oxide.

As alcohols, there can be mentioned lower alcohols such as ethanol, propanol, and isopropanol.

As thickeners, there can be mentioned aqueous polymers including plant polymers such as gum arabic, gum tragacanth, galactan, carob gum, guar gum, carrageenan, pectin, agar and starch (corn, wheat, potato, rice), microbial polymers such as dextran and pullulan, starch polymers such as carboxymethyl cellulose and methylhydroxypropyl starch, animal polymers such as collagen, casein and gelatin, cellulose polymers such as methylcellulose, nitrocellulose, ethylcellulose, hydroxyethyl cellulose, cellulose sodium sulfate, hydroxypropyl cellulose, carboxymethyl cellulose and crystalline cellulose, alginate polymers such as sodium alginate and alginate propylene glycol ester, vinyl polymers such as polyvinylmethyl ether and carboxyvinyl polymer, POE polymers, POE-POP copolymers, acrylic polymers such as sodium polyacrylate and polyacrylate amide, inorganic aqueous polymers such as polyethyleneimine, cation polymer, bentonite, aluminum magnesium silicate, laponite, hectorite and silicic acid anhydride.

As chelating agents, there can be mentioned citramalic acid, agaric acid, glyceric acid, shikimic acid, hinokitiol, gallic acid, tannic acid, caffeic acid, ethylenediamine tetraacetic acid, ethyleneglycol tetraacetic acid, diethylenetriamine pentaacetic acid, phytic acid, polyphosphoric acid, and metaphosphoric acid, as well as derivatives, alkali metal salts, and carboxylates thereof.

As UV absorbing agents, there can be mentioned benzoic acid type UV absorbants such as p-aminobenzoic acid; anthranilic acid type UV absorbants such as methyl anthranilate; salicylic acid type UV absorbants such as octyl salicylate; cinnamic acid type UV absorbants such as isopropyl p-methoxycinnamate and octyl p-methoxycinnamate.

As moisturizing agents, there can be mentioned polyethylene glycol, propylene glycol, dipropylene glycol, 1,3-butyleneglycol, glycerin, diglycerin, xylitol, maltitol, maltose, D-mannitol, glucose, fructose, sodium chondroitin sulfate, sodium hyaluronate, sodium lactate, glucosamine and cyclodextrin.

As medicinal components, there can be blended vitamins such as vitamin A oil, retinol, retinol palmitate, pyridoxine hydrochloride, benzyl nicotinate, nicotinamide, dl-α-tocopheryl nicotine, magnesium ascorbyl phosphate, vitamin D2, dl-α-tocopherol, pantothenic acid, and biotin; anti-inflammatory agens such as azulene and glycyrrhizin; skin-whitening agents such as arbutin, potassium 4-methoxysalicylate, 2-O-ethylascorbic acid and glucoside ascorbate, hormones such as estradiol; astringents such as zinc oxide and tannic acid; fresheners such as L-menthol and camphor; and lysozyme chloride, pyridoxine hydrochloride, and sulfur. Further, it is possible to blend various types of extracts having various medicinal effects. Houttuynia cordata extract, phellodendron bark extract, licorice root extract, herbaceous peony extract, moutan bark extract, sponge cucumber extract, strawberry geranium extract, eucalyptus extract, clove extract, marronnier extract, cornflower extract, seaweed extract, and thyme extract.

As preservatives, there can be mentioned p-oxybenzoic acid esters such as methylparaben, ethylparaben and butylparaben, benzoic acid, salicylic acid, sorbic acid, p-chlorometacresol, hexachlorophen, benzalkonium chloride, chlorhexidine chliride, trichlorocarbanilide, photosensitive elements, phenoxyethanol, isomethyl thiazoline, and the like.

As neutralizing agents, there can be mentioned 2-amino-2-methyl-1-propanol, 2-amino-2-methyl-1,3-propanediol, potassium hydroxide, potassium hydroxide, triethanolamine, and sodium carbonate.

As pH regulating agents, there can be mentioned lactic acid, citric acid, glycolic acid, succinic acid, tartaric acid, malic acid, sodium bicarbonate and ammonium bicarbonate.

As antioxidants, there can be mentioned ascorbic acid, α-tocopherol and carotinoid.

The above components are merely illustrative and the present invention is not limited to them. Also, these components may be combined as appropriate according to the regimen suitable for the desired form.

The formulation of a moisturizing agent and an external composition for skin of the present invention are not specifically limited, and may take any dosage form such as a solution system, a solubilized system, an emulsion system, a powder dispersion system, a water-oil bilayer system, a water-oil-powder three-layer system, an ointment, a gel, an aerosol etc. The form used is not limited, either, and may take the form of a lotion, a milky lotion, a cream, an essence, a gelly, a gel, an ointment, a pack, a mask, a foundation etc.

The moisturizing agent and the external composition for skin of the present invention may be applied to the skin to use in the beauty method for facilitating the moisture retention of the skin, the prevention of wrinkle formation, and/or the reduction and disappearance of wrinkles. The dosage and administration of the moisturizing agent and external composition for skin of the present invention in such beauty methods may not be specifically limited, and may be determined as appropriate depending on the formulation and the state of wrinkles on the skin. Typically, for example 0.1 ml to 1 ml per square centimeters may be rubbed directly on the skin several times per day, for example 1-5 times per day, or a suitable amount may be soaked into gauge etc. and then may attached on the skin.

### Examples

The present invention will now be explained in further detail with reference to specific examples. It should be noted, however, that the present invention is not limited to them in any way.

### [Synthetic example of L-lysyl-β-alanine]

2.2 g of N,N'-dibenzyloxycarbonyl-L-lysine was dissolved in 40 ml of methylene chloride, to which 1.9 g of β-alaninebenzylester tosylate, 1.5 ml of triethylamine, 1.0 g of 1-ethyl-3-(3-dimethylaminopropyl)-carbodiimide hydrochloride, and 0.8 g of 1-hydroxybenzotriazole were added at room temperature, and then stirred for 3 hours. After the mixture was diluted in 100 ml of methylene chloride and washed with 50 ml of purified water, 50 ml of 2M hydrochloric acid, and 50 ml of saturated saline, it was dried in anhydrous magnesium sulfate, filtered, and concentrated. To the residue obtained, 200 ml of methanol was added, 0.5 g of Pd-C (5%, 50% wet) was added, stirred under a hydrogen atmosphere at room temperature for 2 hours, and filter-concentrated to obtain 1.1 g (96%) of the desired product.

### [Test on the effect of moisturizing the skin horny layer]

Changes in the water content of the horny layer on the back of a hairless mouse (HR-1: Hoshino Experiment Animals (HOSHINO JIKKEN DOBUTSU)) by drug application were measured. The test was conducted at n=5, and the water content of the horny layer was measured using the skin conductor (ASA-MX). After determining the water content of the horny layer, 100 µl each of Example 1, and Comparative Examples 1 to 5 of which compositions are shown hereinbelow was applied at the measured site on the back of the mouse once per day. After applying for two consecutive days, the water content of the horny layer was measured on day 3 after the start of the test. They were further applied for two consecutive days, and on day 5 after the start of the test, the water content of the horny layer was measured.

### Example 1

| | |
|---|---|
| L-lysyl-β-alanine | 5.0% by weight |
| 1N hydrochloric acid | To adjust pH 7.5 |
| Alcohol for cosmetics | 50.0 |
| Purified water | balance |

| Comparative Example 1 | |
|---|---|
| Alcohol for cosmetics | 50.0% by weight |
| Purified water | balance |

| Comparative Example 2 | |
|---|---|
| Glycerin | 5.0% by weight |
| Alcohol for cosmetics | 50.0% |
| Purified water | balance |

| Comparative Example 3 | |
|---|---|
| β-alanine | 5.0% by weight |
| Alcohol for cosmetics | 50.0% |
| Purified water | balance |

| Comparative Example 4 | |
|---|---|
| L-lysine hydrochloride | 5.0% by weight |
| Alcohol for cosmetics | 50.0 |
| Purified water | balance |

| Comparative Example 5 | |
|---|---|
| L-lysine-hydroxyproline | 5.0% by weight |
| 1N hydrochloric acid | To adjust pH 7.5 |
| Alcohol for cosmetics | 50.0 |
| Purified water | balance |

The result of changes in water content (%) with the water content before application being set at 100% is shown in the following table.

It was confirmed that the moisturizing agent and the external composition for skin of the present invention can increase the water content of the horny layer on the skin, have an excellent moisturizing effect and a high persistence of moisture retention. Furthermore, it was confirmed that the moisturizing agent of the present invention has a more excellent effect than glycerin that is conventionally widely used as the moisturizing agent. It was further confirmed that the moisturizing agent of the present invention has a more excellent effect than amino acids such as β-alanine and L-lysine, and a more excellent effect than L-lysyl-hydroxyproline, a dipeptide, having lysine as a constituent component.

**Table 1. Result of changes in water content (%) in the horny layer**

| | Drug blended | Day 2 post-application | Day 4 post-application |
|---|---|---|---|
| Ex. 1 | L-lysyl-β-alanine | 147.4 | 163.4 |
| Comp.Ex. 1 | - | 118.7 | 120.7 |
| Comp.Ex. 2 | Glycerin | 114.7 | 121.9 |
| Comp.Ex. 3 | β-alanine | 109.1 | 119.0 |
| Comp.Ex. 4 | L-lysine hydrochloride | 119.3 | 111.6 |
| Comp.Ex. 5 | L-lysyl-hydroxyproline | 109.2 | 112.1 |

### [Stability evaluation]

For L-lysyl-β-alanine hydrochloride (Example 2), L-lysine (Comparative Example 6), and β-alanine (Comparative Example 7), the following external composition for skin was prepared, and the stability thereof was evaluated.

| Lotion | % by weight |
|---|---|
| Ethanol | 3 |
| Glycerin | 2 |
| Drug | 5 |
| 1,3-butylene glycol | 3 |
| POE(60) hydrogenated caster oil | 0.4 |
| Citric acid | 0.03 |
| Citric acid trisodium | 0.07 |
| Edetic acid trisodium | 0.02 |
| Purified water | balance |

### Example 2 L-lysyl-β-alanine hydrochloride

### Comparative Example 6 L-lysine

### Comparative Example 7 β-alanine

50 g each of Example 2, Comparative Example 6 and Comparative Example 7 was placed in a 50 ml screw tube and capped. After storing them in a 50°C incubator for one month, changes in appearance and smell were judged according to the following criteria.

### (Appearance)

⊚: Good (Changes in appearance such as color change, separation, turbidity, crstallization are not observed)
○: Moderately good (Changes are partly seen, but no serious changes)
△: Moderately bad (Changes in appearance such as color change, separation, turbidity, crstallization are partly observed)
×: Bad (Marked changes in appearance such as color change, separation, turbidity, crstallization are observed)

### (Smell)

⊚: Good (No changes)
○: Moderately good (Little changes are observed)
△: Moderately bad (Changes are observed)
×: Bad (Marked changes observed)

### [Table 2]

**Table 2**

| | Appearance | Smell |
|---|---|---|
| Example 2 | ⊚ | ⊚ |
| Comparative Example 6 | ⊚ | ⊚ |
| Comparative Example 7 | ⊚ | × |

The results of the test revealed that the external composition for skin of the present invention having L-lysyl-β-alanine blended therein was excellently stable in terms of smell and appearance. Also the external composition for skin of Comparative Example 6 having β-alanine blended therein was also excellently stable in terms of smell and appearance. On the other hand, for the external composition for skin of Comparative Example 7 having L-lysine blended therein, the stability in appearance was excellent, though its smell deteriorated badly.

The following illustrates the external composition for skin of the present invention and external compositions for skin as the formulation examples of a moisturizing agent. All the external compositions for skin exhibit an excellent effect of moisture retention.

### Formulation example 1: Cream

| | % by weight |
|---|---|
| Stearic acid | 5.0 |
| Stearyl alcohol | 4.0 |
| Isopropyl myristate | 18.0 |
| Glycerin monostearate | 3.0 |
| Propylene glycol | 10.0 |
| L-lysyl-β-alanine monohydrochloride | 3.0 |
| Caustic potash | 0.2 |
| Sodium hydrogensulfite | 0.01 |
| Preservative | q.s. |
| Perfume | q.s. |
| Ion-exchanged water | balance |

### (Preparation method)

L-lysyl-β-alanine monohydrochloride, propylene glycol, and caustic potash are added and dissolved in ion-exchanged water, heat-dissolved and maintained at 70°C (aqueous phase). The other components are mixed and heat-melted, and maintained at 70°C (oily phase). The oily phase is gradually added to the aqueous phase, and after the addition is complete, the temperature is maintained for a while to cause the reaction. Then it is homogeneously emulsified with a homomixer, and it is cooled while stirring well to 30°C to obtain a cream.

### Formulation example 2: Cream

| | % by weight |
|---|---|
| Stearic acid | 6.0 |
| Sorbitan monostearate | 2.0 |
| Polyoxyethylene (20 moles) sorbitan monostearate | 1.5 |
| Propylene glycol | 10.0 |
| L-lysyl-β-alanine dihydrochloride | 7.0 |
| Glycerin trioctanoate | 10.0 |
| Squalene | 5.0 |
| Sodium hydrogensulfite | 0.01 |
| Ethyl parabene | 0.3 |
| Perfume | q.s. |
| Ion-exchanged water | balance |

### (Preparation method)

L-lysyl-β-alanine dihydrochloride and propylene glycol are added and dissolved in ion-exchanged water, heat-dissolved and maintained at 70°C (aqueous phase). The other components are mixed and heat-melted, and maintained at 70°C (oily phase). The oily phase is added to the aqueous phase and preemulsified. After it is homogeneously emulsified with a homomixer, it is cooled while stirring well to 30°C to obtain a cream.

### Formulation example 3: Cream

| | % by weight |
|---|---|
| Stearyl alcohol | 7.0 |
| Stearic acid | 2.0 |
| Hydrogenated lanolin | 2.0 |
| Squalane | 5.0 |
| 2-octyldodecyl alcohol | 6.0 |
| Polyoxyethylene (25 moles) cetyl alcohol ehter | 3.0 |
| Glycerin monostearate | 2.0 |
| Propylene glycol | 5.0 |
| DL-lysyl-β-alanine monohydrochloride | 0.001 |
| Perfume | q.s. |
| Sodium hydrogensulfite | 0.03 |
| Ethyl parabene | 0.3 |
| Ion-exchanged water | balance |

### (Preparation method)

DL-lysyl-β-alanine monohydrochloride and propylene glycol are added and dissolved in ion-exchanged water, heat-dissolved and maintained at 70°C (aqueous phase). The other components are mixed and heat-melted, and maintained at 70°C (oily phase). The oily phase is added to the aqueous phase and preemulsified. After it is homogeneously emulsified with a homomixer, it is cooled while stirring well to 30°C to obtain a cream.

### Formulation example 4: Emulsion

| | % by weight |
|---|---|
| Stearic acid | 2.5 |
| Cetyl alcohol | 1.5 |
| Vaseline | 5.0 |
| Liquid paraffin | 10.0 |
| Polyoxyethylene (10 moles) monooleate | 2.0 |
| Polyethylene glycol 1500 | 3.0 |
| Triethanolamine | 1.0 |
| D-lysyl-β-alanine | 10.0 |
| Sodium hydrogensulfite | 0.01 |
| Ethyl parabene | 0.3 |
| Carboxy vinyl polymer | 0.05 |
| Perfume | q.s. |
| Ion-exchanged water | balance |

### (Preparation method)

A carboxy vinyl polymer is dissolved in a small amount of ion-exchanged water (A phase). To the rest of the ion-exchanged water, D-lysyl-β-alanine, polyethylene glycol 1500, and triethanolamine are added, heat-dissolved and maintained at 70°C (aqueous phase). The other components are mixed and heat-melted, and maintained at 70°C (oily phase). The oily phase is added to the aqueous phase and preemulsified. After the A phase is homogeneously emulsified with a homomixer, it is cooled while stirring well to 30°C to obtain an emulsion.

### Formulation example 5: Emulsion

| (Oily phase part) | % by weight |
|---|---|
| Stearyl alcohol | 1.5 |
| Squalene | 2.0 |
| Vaseline | 2.5 |
| Deodorized liquid lanolin | 1.5 |
| Evening primrose oil | 2.0 |
| Isopropyl myristate | 5.0 |
| Glycerin monooleate | 2.0 |
| Polyoxyethylene (60 moles) hdrogenated castor oil | 2.0 |
| Tocopherol acetate | 0.05 |
| Ethyl parabene | 0.2 |
| Buthyl parabene | 0.1 |
| Perfume | q.s. |

| (Aqueous phase part) | |
|---|---|
| L-lysyl-β-alanine potassium salt | 1.0 |
| Sodium hydrogensulfite | 0.01 |
| Glycerin | 5.0 |
| Sodium hyaluronate | 0.01 |
| Carboxy vinyl polymer | 0.2 |
| Potassium hydroxide | 0.2 |
| Purified water | balance |

### (Preparation method)

The oily phase part is dissolved at 70°C. The aqueous phase part is dissolved at 70°C, and the oily phase part is mixed with the aqueous phase part. After it is emulsified with an emulsifier, it is cooled to 30°C with a heat exchanger to obtain an emulsion.

### Formulation example 6: Jelly

| | % by weight |
|---|---|
| 95% ethyl alcohol | 10.0 |
| Dipropylene glycol | 15.0 |
| Polyoxyethylene (50 moles) oleyl alcohol ehter | 2.0 |
| Carboxy vinyl polymer | 1.0 |
| Caustic soda | 0.15 |
| L-arginine | 0.1 |
| L-lysyl-β-alanine monoglutamate | 1.0 |
| Methyl parabene | 0.2 |
| Perfume | q.s. |
| Ion-exchanged water | balance |

### (Preparation method)

A carboxy vinyl polymer is homogeneously dissolved in an ion-exchanged water. On the other hand, polyoxyethylene (50 moles) oleylalcohol ether is dissolved in 95% ethanol, and added to the aqueous phase. Then, after adding the other components, it is neutralized with caustic soda and L-arginine, and thickened to obtain a jelly.

### Formulation example 7: Beauty essence

| | % by weight |
|---|---|
| (A phase) | |
| Ethanol(95%) | 10.0 |
| Polyoxyethylene (20 moles) oleyl dodecanol | 1.0 |
| Methyl parabene | 0.15 |
| Panthothenil ethylether | 0.1 |

| (B phase) | |
|---|---|
| Potassium hydroxide | 0.1 |

| (C phase) | |
|---|---|
| Glycerin | 5.0 |
| Dipropylene glycol | 10.0 |
| Sodium hydrogensulfite | 0.03 |
| L-lysyl-β-alanine oxalate | 0.05 |
| Carboxy vinyl polymer | 0.2 |
| Purified water | balance |

### (Preparation method)

The A phase and the C phase are each homogeneously dissolved, and the C phase is added to the A phase to solubilize. Then after adding the B phase thereof, it is filled to obtain an essence.

### Formulation example 8: Pack

| | % by weight |
|---|---|
| (A phase) | |
| Dipropylene glycol | 5.0 |
| Polyoxyethylene (60 moles) hydrogenated castor oil | 5.0 |

| (B phase) | |
|---|---|
| Olive oil | 5.0 |
| Tocopherol acetate | 0.2 |
| Ethyl parabene | 0.2 |
| Perfume | 0.2 |

| (C phase) | |
|---|---|
| L-lysyl-β-alanine succinate | 1.0 |
| Sodium hydrogensulfite | 0.03 |
| Polyvinyl alcohol (saponification number 2000, polymerization degree 2000) | 13.0 |
| Ethanol | 7.0 |
| Purified water | balance |

### (Preparation method)

The A phase, the B phase and the C phase are each homogeneously dissolved, and the B phase is added to the A phase to solubilize. Then after adding this to the C phase, it is filled to obtain a pack.

### Formulation example 9: Ointment

| | % by weight |
|---|---|
| Polyoxyethylene (30 moles) cetyl alcohol | 2.0 |
| Glycerin monostearate | 10.0 |
| Liquid paraffin | 10.0 |
| Vaseline | 40.0 |
| Cetanol | 6.0 |
| Methyl parabene | 0.1 |
| Buthyl parabene | 0.1 |
| Glycerin monostearate | 2.0 |
| L-lysyl-β-alanine monotaurine monohydrochloride | 5.0 |
| Propylene glycol | 10.0 |
| Ion-exchanged water | balance |
| Perfume | q.s. |

### (Preparation method)

Propylene glycol is added to ion-exchanged water, dissolved, and heated and maintained at 70°C (aqueous phase). The other components are mixed and dissolved at 70°C (oily phase). The oily phase is added to the above aqueous phase, homogeneously emulsified with a homomixer, and after cooling, filled to obtain an ointment.

### Formulation example 10: Cream

| | % by weight |
|---|---|
| Liquid paraffin | 8.0 |
| Vaseline | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| Treahalose | 1.0 |
| Tetra 2-ethylhexanoic acid pentaerythrite | 4.0 |
| Polyoxyethylene glyceryl monoisostearate | 2.0 |
| Polyoxyethylen glyceryl monostearate | 1.0 |
| Lipophilic glycerin monostearate | 2.0 |
| Citric acid | 0.05 |
| Sodium citrate | 0.05 |
| Potassium hydroxide | 0.015 |
| Lipophilic licorice extract | 0.1 |
| Retinol palmitate (1 million units) | 0.25 |
| L-lysyl-β-alanine sulfate | 1.0 |
| Tocopherol acetate | 0.1 |
| Paraoxy benzoic acid ester | q.s. |
| Phenoxyethanol | q.s. |
| ibutylhydroxytoluene | q.s. |
| Trisodium edetate | 0.05 |
| 4-t-butyl-4'-methoxybenzoyl methane | 0.01 |
| 2-ethylhexyl paramethoxycinnamate | 0.1 |
| β-carotene | 0.01 |
| Polyvinyl alcohol | 0.5 |
| Hydroxyethyl cellulose | 0.5 |
| Carboxyvinyl polymer | 0.05 |
| Purified water | balance |
| Perfume | q.s. |

### Formulation example 11: Cream

| | % by weight |
|---|---|
| Vaseline | 2.0 |
| Dimethylpolysiloxane | 2.0 |
| Ethanol | 5.0 |
| Behenyl alcohol | 0.5 |
| Batyl alcohol | 0.2 |
| Glycerin | 7.0 |
| 1,3-butylene alcohol | 5.0 |
| Polyethylene glycol 20000 | 0.5 |
| Jojoba oil | 3.0 |
| Squalane | 2.0 |
| Hydroxystearic acid phytosteryl | 0.5 |
| Tetra 2-ethylhexanoic acid pentaerythrite | 1.0 |
| Polyoxyethylene hydrogenated castor oil | 1.0 |
| Potassium hydroxide | 0.1 |
| Sodium pyrosulfite | 0.01 |
| Sodium hexametaphosphate | 0.05 |
| Stearyl glycyrrhetinate | 0.1 |
| Pantothenylethyl ether | 0.1 |
| Arbutin | 7.0 |
| Tranexamic acid methylamide hydrochloride | 11.0 |
| L-lysyl-β-alanine phosphate | 1.0 |
| L-lysyl-β-alanine aspartate | 1.0 |
| Tocopherol acetate | 0.1 |
| Sodium hyaluronate | 0.05 |
| Paraoxy benzoic acid ester | q.s. |
| Trisodium edetate | 0.05 |
| 4-t-butyl-4'-methoxydibenzoyl methane | 0.1 |
| Mono-2-ethylhexanoic acid glyceryl diparamethoxycinnamate | 0.1 |
| Yellow iron oxide | q.s. |
| Xanthan gum | 0.1 |
| Carboxyvinyl polymer | 0.2 |
| Purified water | balance |

### Formulation example 12: Lotion

| | % by weight |
|---|---|
| 1.3-butyleneglycol | 6.0 |
| Glycerin | 4.0 |
| Oleyl alcohol | 0.1 |
| POE(20) sorbitan monolaurate | 0.5 |
| POE(15) laurylalcohol ester | 0.5 |
| Ethanol | 10.0 |
| L-lysyl-β-alanine monolactate | 0.3 |
| Purified water | balance |

### Formulation example 13: Lotion

| | % by weight |
|---|---|
| (Alcohol phase) | |
| Ethanol | 10.0 |
| Oleyl alcohol | 0.1 |
| POE(20) sorbitan monolaurate | 0.5 |
| FOE(15) lauryl ether | 0.5 |
| Preservative | q.s. |
| Perfume | q.s. |

| (Aqueous phase) | |
|---|---|
| L-lysyl-β-alanine betaine salt | 20.0 |
| 1.3-butyleneglycol | 6.0 |
| Glycerin | 4.0 |
| Ion-exchanged water | balance |

### Formulation example 14: Solid powder foundation

| | % by weight |
|---|---|
| Talc | 15.0 |
| Sericite | 10.0 |
| Globular nylon powder | 10.0 |
| Porous silicic anhydride powder | 15.0 |
| Boron nitride | 5.0 |
| Titanium dioxide | 5.0 |
| Iron oxide | 3.0 |
| Zinc stearate | 5.0 |
| L-lysyl-β-alanine tosylate | 1.0 |
| Liquid paraffin | balance |
| Glycerin triisooctanoate | 15.0 |
| Solbitan sesquioleate | 1.5 |
| Preservative | q.s. |
| Perfume | q.s. |

### Formulation example 15: Water-in-oil emulsified foundation

| | % by weight |
|---|---|
| Globular nylon | 10.0 |
| Porous silicic anhydride powder | 8.0 |
| Titanium mica | 2.0 |
| Siliconized sericite | 2.0 |
| Siliconized mica | 12.0 |
| Siliconized titanium dioxide | 5.0 |
| Siliconized iron oxide | 2.0 |
| Ion-exchanged water | balance |
| L-lysyl-β-alanine | 1.0 |
| Decamethyl cyclopentane siloxane | 18.0 |
| Dimethyl polysiloxane | 5.0 |
| Squalane | 1.0 |
| POE-denatured dimethyl polysiloxane | 2.0 |
| Preservative | q.s. |
| Perfume | q.s. |

Similarly to those in Example 1, the external compositions for skin obtained in Formulation examples 1-15 had a good water-retention effect, an excellent stability and sense of use.

## Claims

1. A moisturizing agent comprising one or more than one compound selected from the group consisting of lysyl-β-alanine represented by the following general formula (1) and a salt thereof:

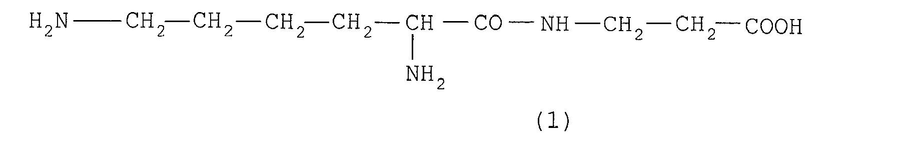

2. An external composition for skin that comprises one or more than one compound selected from the group consisting of the above lysyl-β-alanine compound and a salt thereof.
